Europäisches Patentamt

European Patent Office

Office européen des brevets

(11) Veröffentlichungsnummer: **0 560 057 A1**

# (12) EUROPÄISCHE PATENTANMELDUNG

(21) Anmeldenummer: **93101883.2**

(22) Anmeldetag: **06.02.93**

(51) Int. Cl.5: **C07C 43/04**, C07C 41/42, C07C 41/09

(30) Priorität: **13.03.92 DE 4208028**

(43) Veröffentlichungstag der Anmeldung:
**15.09.93 Patentblatt 93/37**

(84) Benannte Vertragsstaaten:
**AT BE CH DE DK ES FR GB IT LI LU NL**

(71) Anmelder: **LEUNA-WERKE AG**
**Am Haupttor**
**D-06236 Leuna(DE)**

(72) Erfinder: **Holm, Rainer, Dr.**
**Azaleenstrasse 52**
**O-4090 Halle(DE)**
Erfinder: **Kohl, Günter, Dr.**
**Haydn-Strasse 6**
**O-4850 Weissenfels(DE)**
Erfinder: **Veit, Joachim, Dl.**
**Bl. 643/7**
**O-4090 Halle(DE)**
Erfinder: **Daute, Ralf, Dr.**
**Burger Hof 11**
**O-4090 Halle(DE)**
Erfinder: **Mohr, Klaus, DC.**
**Stolberger Strasse 16**
**O-4020 Halle(DE)**

(54) **Verfahren zur Herstellung von hochreinem Dimethylether.**

(57) Bei der Erzeugung von hochreinem Dimethylether in Aerosolqualität kommt es bei der Verwendung von Niederdruckmethanol wiederholt zu Geruchsirritationen, als deren Ursache Amine ermittelt wurden. Daher soll das Produkt durch eine unkomplizierte Destillationstechnologie aus Methanoldehydratisierungsprodukten wechselnder Zusammensetzung in stabiler, geruchsfreier Qualität erzeugt werden.

Nach der Dehydratisierung und Zuführung des Dehydratisierungsproduktes in ein Destillationssystem wird das genannte Problem gelöst, indem in einer 1. Kolonne eine Vorlauffraktion von 0,5 bis 5 Masse% vom Kolonneneinlaufprodukt als Kopfprodukt abgenommen wird, wobei oberhalb des Einlaufbodens dieser 1. Kolonne eine Waschflüssigkeit, bestehend aus dem Sumpfprodukt einer 2. Kolonne, die neben Methanol und Wasser eine Reihe höhersiedender Verunreinigungen enthält, aufgegeben wird, und in der 2. Kolonne, in die das Sumpfprodukt der 1. Kolonne zugeführt wird, der hochreine Dimethylether über Kopf von den höhersiedenden Komponenten im Sumpf abgetrennt wird.

EP 0 560 057 A1

Die Erfindung betrifft ein Verfahren zur Herstellung von hochreinem Dimethylether durch Dehydratisierung von Methanol an einem Aluminiumoxidkatalysator und nachfolgende destillative Abtrennung von geruchsbildenden Verunreinigungen. Der hochreine Dimethylether erfüllt die wachsenden Reinheitsanforderungen der Aerosol- und Kosmetikindustrie.

Dimethylether wurde bis zur Abstellung der Hochdruck (HD)-Methanolsynthesen als Nebenprodukt dieser Anlagen in Mengen bis zu 5 % des Syntheseanfallproduktes erzeugt und durch Destillation aus den Vorläufen der Rohprodukte gewonnen. Nach der Einführung der Niederdruck (ND)-Methanolanlagen nahm die Verbreitung der HD-Methanolanlagen stark ab. In den ND-Methanolsyntheseanlagen spielt die Erzeugung des Dimethylethers infolge sehr hoher Selektivitäten der Methanolerzeugung keine Rolle.

Zur Herstellung von Dimethylether durch Dehydratisierung von Methanol sind schon weit vor dem Abstellungszeitraum der HD-Methanolsyntheseanlagenzahlreiche Verfahren beschrieben worden.

Eine allgemeine Herstellungsmethode zur Erzeugung von aliphatischen Ethern durch Erhitzen von Alkoholen in Gegenwart von Zinkchlorid wird in DE 680 328 beansprucht. Nach den Patentanmeldungen GB 332 756, GB 350 010, GB 403 402, US 1 873 537, FR 701 335, JP 56-40654 sind zur Umwandlung des Methanols eine Vielzahl acider Verbindungen, wie Eisenchlorid, Kupfersulfat, Zinkchlorid, Manganchlorid, Aluminiumchlorid und -sulfat, Chromsulfat, Alaune, Thoriumverbindungen, Aluminiumoxid, Titanoxid, Silicagel oder Aluminiumphosphat geeignet. In der neueren Patentliteratur spielt besonders die Anwendung von Aluminiumsilikaten oder -titanaten zur Dehydratisierung des Methanols eine Rolle. Hier wird in EP 099 676, EP 270 852 und EP 124 078 dem Verhältnis des Aluminiumoxids zum Siliziumdioxid die besondere Bedeutung zur Erreichung hoher Aktivitäten und Selektivitäten zugesprochen oder durch die Titanatanwendung in US 4 595 785 eine verminderte Koksbildung erreicht.

Desweiteren dienen auch Verfahren zur gekoppelten Herstellung von Methanol und Dimethylether oder nur Dimethylether aus Synthesegasgemischen von $CO/CO_2/H_2$ zur Dimethylethererzeugung. Nach DE 2 362 944, DE 2 757 788, EP 164 156, EP 324 475, NL 179 647, JP 86-043332, JP 89-009303 und DD 291 937 werden Kupfer-, Zinkoxid-, Chromoxid- und Aluminiumoxidträgersysteme zur Methanol- und/oder Etherbildung mit dehydratisierend wirkenden Komponenten gekoppelt und eingesetzt.

Der Dimethylether wurde und wird großtechnisch vorwiegend für die Herstellung von Dimethylsulfat verwendet. Bei dieser Anwendung spielt als Qualitätskriterium hauptsächlich der Wassergehalt eine Rolle. In den letzten Jahren verschiebt sich das Gewicht der Anwendung des Dimethylethers in die Aerosolindustrie. Mit der Verwendung im Aerosolsektor werden an die Reinheit des Dimethylethers höhere Anforderungen gestellt, besonders was die Geruchsfreiheit des Produktes und den Gehalt an toxischem Methanol betrifft.

Das Ziel der erhöhten Reinheit des Dimethylethers wird durch verschiedene Verfahren erreicht. Nach EP 270 852 wird spezifikationsgerechtes Methanol der Dehydratisierung unterworfen und das Dehydratisierungsgemisch in einer Kolonne getrennt, in der neben dem Kopfprodukt von Gasen und Dimethylether in zwei Seitenstromabnahmen der reine Dimethylether und eine Verunreinigungen enthaltende, zwischen Dimethylether und Methanol siedende Zwischenfraktion entnommen wird. Die beschriebene Kolonnenführung löst zwar die gestellte Aufgabe, aber im technischen Betrieb ist bekannt, daß mit schwankenden Betriebszuständen und Einsatzgemischen eine solche Kolonne nur sehr schwierig zu betreiben ist und ein hoher Analysen- und Regelaufwand zur Gewährleistung der geforderten Dimethyletherreinheiten bei quantitativer Etherabtrennung notwendig ist. Bei der Gewinnung des Dimethylethers im Seitenstrom ist analytisch nachweisbar, daß in diesem Seitenstrom im geringen Umfang leichtersiedende Verunreinigungen, insbesondere Methylamine und Kohlenwasserstoffe, vorhanden sind. Damit in Verbindung stehen mehr oder weniger starke Geruchsintensitäten in Abhängigkeit von der Qualität des Syntheseeinsatzproduktes.

Die Ökonomie der Dimethylethererzeugung und -abtrennung wird nach DE 3 817 816 dahingehend verbessert, daß das Syntheseprodukt einer ND-Methanolsyntheseanlage ohne Totalkondensation und Abtrennung des Synthesegases unter Nutzung der Kondensations- und Abwärme der Synthesen direkt der destillativen Aufarbeitung zugeführt wird. Als zusätzlicher Aufwand haftet diesem Verfahren die Rückführung und notwendige Kompressionsarbeit für das Methanolsynthesegas an. Desweiteren ist bekannt, daß auch die sehr selektiv arbeitende ND-Methanolsynthese nicht frei von Nebenreaktionen ist und bei der beschriebenen Lösung diese Nebenprodukte der Methanolbildung, wie Amine, Methylformiat, Aceton, Methylethylketon, Ameisensäureethyl- bis -butylester und Essigsäureester sowie besondere Kohlenwasserstoffe die Abtrennung des Dimethylethers erschweren und zu Geruchsbelästigungen führen können. Außerdem enthält auch das ungereinigte ND-Methanol aus der spezifischen Syntheseführung in Gegenwart von $CO_2$ im Synthesegas einen Teil an Reaktionswassern, welches den Umsatz in der Dehydratisierungsstufe bremst.

In DD 282 909 wird ein Verfahren zur Gewinnung von reinem Dimethylether durch katalytische Dehydratisierung von Methanol und Zuführung des Dehydratisierungsproduktes aus einem Dimethylethersynthesereaktor in eine Destillationskolonne zur Gewinnung von reinem Dimethylether an bestimmten Böden dieser Kolonne und Entnahme des reinen Dimethylethers und einer Verunreinigungen enthaltenden Fraktion an bestimmten Böden der gleichen Kolonne, wobei zusätzlich zum Dehydratisierungsprodukt eine Waschflüssigkeit und Basen eingesetzt werden können, beschrieben.

Die Lösung hat den Vorteil, den Reindimethylether in einer Trennkolonne zu gewinnen, wobei die Qualitätskontrolle für die Anwendung des Dimethylethers in der Aerosolindustrie durch eine subjektive Geruchsschwellenfestlegung mit sensorischer Kontrolle durch geübte Personen erfolgt. Ein Nachteil der beschriebenen Lösung besteht in der durch die Seitenstromabnahme des Reindimethylethers bedingten Anwesenheit endlicher Gehalte an leichtflüchtigen Verunreinigungen in diesem Seitenstrom, der mit wechselnden Rohstoffqualitäten mehr oder weniger schwankt.

Das Methanol aus einem ND-Methanolsynthesekomplex, der hochsiedende Rückstände der Erdölverarbeitung stark schwankender Zusammensetzung hinsichtlich von Metallen sowie S- und N-enthaltenden Verbindungen zur Synthesegaserzeugung verwendet, wobei die Rohstoffverunreinigungen auch noch die Gasreinigungsstufe belasten, ist von bestimmten Aminspurengehalten wechselnder Konzentration betroffen. Auch nach der Inbetriebnahme neuer Kontakte erhält man ein sogenanntes "Amin"-Methanol. Die wechselnden Aminspurengehalte beeinträchtigen normalerweise die Methanolqualität für die konventionellen Anwendungsfälle nicht.

Bei der Erzeugung von hochreinem Dimethylether in Aerosolqualität kam es nun bei Anwendung der genannten Trennverfahren wiederholt bei der Verwendung des, wie oben erläutert, erzeugten Methanols zu Geruchsirritationen, als deren Ursachen mit Hilfe stärkster Anreicherung über GC/MS-Kopplung Di-/Trimethylamine ermittelt wurden. Nach den bekannten Siedepunkten sollten diese Amine bei der destillativen Trennung des Dimethylethers bei den höhersiedenden Nebenprodukten verbleiben. Azeotrope Gemische der Hauptkomponenten oder der identifizierten Nebenprodukte mit den Aminen sind nicht bekannt. Außerdem ist es praktisch sehr schwierig durchführbar, immer das geübte Team zur Geruchsschwellenbestimmung hinzuzuziehen. Die zusätzlich vorgeschlagene Wäsche des Kopfproduktes der Kolonne führt außerdem mögliche geruchsbildende Verunreinigungen in diese zurück und schleust sie in den als Seitenstrom gewonnenen Reindimethylether ein.

Die bekannten technischen Lösungen richten ihre Aufmerksamkeit auf die Auskreisung von Zwischensiedern, die oberhalb des Dimethylethers und unterhalb des Methanols sieden. Sie geben keine Lösung für die gezielte Ausschleusung von leichtersiedenden Verunreinigungen oder Nebenprodukten an, deren Destillationsverhalten nicht bekannt ist, durch deren Auskreisung aber die Geruchsmerkmale des reinen Dimethylethers bestimmt werden.

Es bestand somit das Problem, einen hochreinen Dimethylether (DME) durch eine unkomplizierte Destillationstechnologie aus Methanoldehydratisierungsprodukten wechselnder Zusammensetzung in stabiler, geruchsfreier Qualität zu erzeugen.

Dieses Problem der Herstellung von hochreinem Dimethylether, der insbesondere frei von aminischen Verunreinigungen ist, wird durch Dehydratisierung von Methanol an einem Aluminiumoxid- oder Alumosilikatkatalysator im Temperaturbereich von 200 bis 450 °C und einem Druckbereich von 0,1 bis 25 MPa und Zuführung des Dehydratisierungsproduktes in ein Destillationssystem erfindungsgemäß dadurch gelöst, daß in einer 1. Kolonne eine Vorlauffraktion von 0,5 bis 5 Masse%, vorzugsweise 1 Masse%, vom Kolonneneinlaufprodukt als Kopfprodukt abgenommen wird, wobei oberhalb des Einlaufbodens dieser 1. Kolonne eine Waschflüssigkeit, bestehend aus dem Sumpfprodukt einer 2. Kolonne, die neben Methanol und Wasser eine Reihe höhersiedender Verunreinigungen enthält, aufgegeben wird, und in der 2. Kolonne, in die das Sumpfprodukt der 1. Kolonne zugeführt wird, der hochreine Dimethylether über Kopf von den höhersiedenden Komponenten im Sumpf abgetrennt wird, wobei das Sumpfprodukt der 2. Kolonne als Waschflüssigkeit 1 bis 20 Böden, vorzugsweise 5 bis 10 Böden, oberhalb des Einlaufbodens der 1. Kolonne zugeführt wird und zwar in Mengen von 1 bis 50 Masse%, vorzugsweise 5 bis 20 Masse%, bezogen auf das Einsatzprodukt der 1. Kolonne, sowie die Rücklaufverhältnisse in der 1. Kolonne bei 10 bis 100, vorzugsweise bei 30 bis 60, und für die 2. Kolonne bei 0,5 bis 10, vorzugsweise bei 1 bis 5, liegen.

Es wurde überraschenderweise gefunden, daß im Gegensatz zu vorgenannten Verfahrensweisen eine vollständige Abtrennung vor allem der im Vergleich zu DME an sich höhersiedenden Methylamine neben anderen Leichtsiedern über Kopf einer ersten Kolonne dann gelingt, wenn in die Obersäule dieser Kolonne zusätzlich ein Produkt eingespritzt wird, das neben Methanol und Wasser die im Syntheseprodukt vorhandenen höhersiedenden und zumeist unbekannten Nebenprodukte (x-Komponenten) enthält. Um eine vollständige Beseitigung der über Kopf abtrennbaren Verunreinigungen vom Dimethylether zu erreichen, ist dann allerdings eine 2-Kolonnenfahrweise erforderlich, die eine Seitenstromentnahme des Rein-Dimethylethers aus der Obersäule und damit dessen Verunreinigung mit Spuren leichtersiedender Komponenten vermeidet.

Zum Einsatz in die Dehydratisierungsstufe gelangt ein ND-Methanol, welches im bestimmungsgemäßen Betrieb höchstens 10 ppm Ethanol, 1 ppm Wasser, 30 ppm Ketone und 0,1 bis 5 ppm N-haltige Verbindungen enthält. Die Dehydratisierung wird vorzugsweise bei 1 bis 2 MPa an Aluminiumoxid- oder Alumosilikatkatalysatoren bei erhöhten Temperaturen durchgeführt.

Das Dehydratisierungsprodukt wird nach der Umsetzung flüssig abgeschieden. Die Dehydratisierungsbedingungen können in weiten Grenzen variiert werden. Sie richten sich nach den Aktivitätszuständen der Dehydratisierungskontakte und den Abscheideverhältnissen für das flüssige Produktgemisch.

In Abhängigkeit von wechselnden Anteilen an x-Komponenten im Einsatzmethanol und schwankenden Konzentrationen von Spurenverunreinigungen N-enthaltender Verbindungen hat es sich im Zusammenhang mit Parameterschwankungen in der Dehydratisierungsstufe ergeben, daß eine stabile geruchsfreie Dimethyletherqualität in der 2. Kolonne erhalten wird, wenn 0,5 bis 5 Masse%, im bestimmungsgemäßen Betrieb vorzugsweise 1 Masse%, vom Einsatzprodukt gerechnet, als Vorlauf in der ersten Kolonne abgetrennt werden und zugleich nach dem erfindungsgemäßen Verfahren eine Aufgabe des Sumpfproduktes der 2. Kolonne 1 bis 20 Böden oberhalb des Einlaufs der 1. Kolonne in Mengen von 1 bis 50 Gewichtsprozent des Sumpfproduktes, bezogen auf das Einsatzprodukt der 1. Kolonne erfolgt.

Der Aufgabeboden für das Synthesegasprodukt in die 1. Kolonne ist in weiten Grenzen variierbar, sollte aber vorteilhafterweise 1 bis 10 Böden über der Sumpfblase liegen. Die Menge des zugesetzten Sumpfproduktes der 2. Kolonne in die Obersäule der 1. Kolonne richtet sich nach dem Gehalt an höhersiedenden x-Komponenten im Sumpf der 2. Kolonne und nach dem Gehalt an aminischen Verbindungen im Syntheseprodukt. Im allgemeinen bedingen geringere Anteile an höhersiedenden x-Komponenten und höhere Amingehalte im Syntheseprodukt eine größere Rückführungsmenge. Im erfindungsgemäßen Verfahren wird eine Rückführungsmenge von 1 bis 50 Masse%, vorzugsweise 5 bis 20 Masse%, bezogen auf das eingesetzte Syntheseprodukt, als ausreichend angesehen.

An die Qualität des erfindungsgemäß zurückgeführten Sumpfproduktes der 2. Kolonne werden keine besonderen Anforderungen gestellt. Es hat sich gezeigt, daß offenbar nicht die prozeßbedingte Methanol/Wasser-Verteilung sondern die Konzentrationen der x-Komponenten die quantitative Abtrennung der Amine im Kopfprodukt der 1. Kolonne bewirkt.

Die Rücklaufverhältnisse in beiden Kolonnen richten sich nach der gewünschten Reinheit des Rein-Dimethylethers und, in Abhängigkeit davon, in der 1. Kolonne nach dem Gehalt an leichtersiedenden Verunreinigungen, in der 2. Kolonne nach dem Gehalt an schwerer als DME siedenden Komponenten.

Beim erfindungsgemäßen Verfahren haben sich für die 1. Kolonne Rücklaufverhältnisse von 10 bis 100, vorzugsweise 30 bis 60, für die 2. Kolonne Rücklaufverhältnisse von 0,5 bis 10, vorzugsweise 1 bis 5, als ausreichend erwiesen.

Nachfolgend wird das erfindungsgemäße Verfahren an einem Beispiel erläutert und mit einem Verfahren nach dem Stand der Technik verglichen.

Beispiel 1
(Zusammensetzungen A bis I in Tabelle 1).

Einer Kolonne mit 28 Ventilböden wurden auf den 20. Boden (vom Kopf der Kolonne) 1000 kg/h eines Synthesegemisches der Zusammensetzung A zugeführt. Auf den 10. Boden von oben wurden 100 kg Waschflüssigkeit der Zusammensetzung E aufgegeben. Der Destillationsdruck lag bei 1,2 MPa, das Rücklaufverhältnis bei 40. Als Kopfprodukt wurden 10 kg/h mit der Zusammensetzung B, als Sumpfprodukt 1090 kg/h mit der Zusammensetzung C gewonnen.

In eine zweite Kolonne mit 30 Ventilböden wurden auf den 20. Boden von oben 1000 kg/h Sumpfprodukt der ersten Kolonne mit der Zusammensetzung C eingespritzt. Bei einem Druck von 1,2 MPa und einem Rücklaufverhältnis von 2 wurden 550 kg/h Kopfprodukt mit der Zusammensetzung D und 450 kg/h Sumpfprodukt mit der Zusammensetzung E erhalten. Im Kopfprodukt waren neben 3 ppm Methanol und 2 ppm Kohlenwasserstoffe (KW-Stoffe) keine weiteren Verunreinigungen nachweisbar. Störende Gerüche konnten gleichfalls nicht festgestellt werden.

Beispiel 2 (Vergleichsbeispiel)

Einer Kolonne mit 60 Ventilböden wurden am 45. Boden von oben 1000 kg/h eines Gemisches der Zusammensetzung A zugeführt. Auf den 20. Boden von oben wurden 100 kg/h einer Waschflüssigkeit aus 45 Masse% Methanol und 55 Masse% Wasser aufgegeben. Am Kopf der Kolonne wurden 10 kg/h eines Produktes der Zusammensetzung H, vom 25. Boden 20 kg/h eines Seitenstromes der Zusammensetzung G abgenommen. Vom 7. Boden von oben wurden als Seitenstrom 520 kg/h Dimethylether der Zusammenset-

4

zung F und am Sumpf 560 kg/h ein Produkt der Zusammensetzung I gewonnen. Der Destillationsdruck betrug in diesem Fall 1,2 MPa, das Rücklaufverhältnis 40.

Der gewonnene Dimethylether besaß einen schwach unangenehmen Geruch, es wurden 2 ppm Methylamine neben 1 ppm Methan und 5 ppm C2-C4-KW-Stoffen nachgewiesen.

Tabelle 1

| | DME (Masse%) | MeOH+ (Masse%) | $H_2O$ (Masse%) | $CO_2$/Inerte (ppm) | $CH_4$ (ppm) | KW-Stoffe $C_2$-$C_4$ (ppm) | Methyl-amine (ppm) | $\sum x_1$ [1] (ppm) | Formiat (ppm) | $\sum x_2$ [2] (ppm) |
|---|---|---|---|---|---|---|---|---|---|---|
| A | 55,1 | 19,9 | 24,7 | 1800 | 100 | 60 | 5 | 40 | 10 | 1000 |
| B | 99,49 | 0,01 | nn | 4000 | 1000 | 600 | 20 | 200 | nn | 10 |
| C | 55,2 | 19,95 | 24,7 | nn | nn | 2 | nn | 20 | 10 | 1200 |
| D | 99,999 | 0,0002 | nn | nn | nn | 2 | nn | 10 | nn | nn |
| E | 0,05 | 44,5 | 55,2 | nn | nn | 10 | nn | nn | 20 | 2500 |
| F | 99,99 | 0,001 | nn | nn | 1 | 5 | 2 | 200 | nn | nn |
| G | 95,9 | 1,25 | 0,74 | nn | 10 | 1000 | 2 | 40 | 100 | 20000 |
| H | 99,2 | 0,06 | nn | 6500 | 500 | 500 | 18 | 200 | nn | nn |
| I | 0,07 | 45,2 | 54,7 | nn | nn | 1 | nn | nn | 8 | 100 |

[1] $x_1$ nach GC vor DME
[2] $x_2$ nach GC nach DME
nn - nicht nachweisbar

**Patentansprüche**

1.  Verfahren zur Herstellung von hochreinem Dimethylether, der insbesondere frei von aminischen Verunreinigungen ist, durch Dehydratisierung von Methanol an einem Aluminiumoxid- oder Alumosilikatkatalysator im Temperaturbereich von 200 bis 450 °C und einem Druckbereich von 0,1 bis 25 MPa und Zuführung des Dehydratisierungsproduktes in ein Destillationssystem, dadurch gekennzeichnet, daß in einer 1. Kolonne eine Vorlauffraktion von 0,5 bis 5 Masse%, vom Kolonneneinlaufprodukt als Kopfprodukt abgenommen wird, wobei oberhalb des Einlaufbodens dieser 1. Kolonne eine Waschflüssigkeit, bestehend aus dem Sumpfprodukt einer 2. Kolonne, die neben Methanol und Wasser eine Reihe höhersiedender Verunreinigungen enthält, aufgegeben wird, und in der 2. Kolonne, in die das Sumpfprodukt der 1. Kolonne zugeführt wird, der hochreine Dimethylether über Kopf von den höhersiedenden Komponenten im Sumpf abgetrennt wird.

2.  Verfahren nach Anspruch 1, dadurch gekennzeichnet, daß in der 1. Kolonne eine Vorlauffraktion von 1 Masse% vom Kolonneneinlaufprodukt als Kopfprodukt abgenommen wird.

3.  Verfahren nach Anspruch 1 und 2, dadurch gekennzeichnet, daß als Waschflüssigkeit das Sumpfprodukt der 2. Kolonne 1 bis 20 Böden, vorzugsweise 5 bis 10 Böden, oberhalb des Einlauf der 1. Kolonne zugeführt wird.

4.  Verfahren nach Anspruch 1 bis 3, dadurch gekennzeichnet, daß als Waschflüssigkeit in die 1. Kolonne 1 bis 50 Masse%, vorzugsweise 5 bis 20 Masse%, Sumpfprodukt der 2. Kolonne, bezogen auf das Einsatzprodukt der 1. Kolonne, eingesetzt werden.

5.  Verfahren nach Anspruch 1 bis 4, dadurch gekennzeichnet, daß die Rücklaufverhältnisse in der 1. Kolonne bei 10 bis 100, vorzugsweise bei 30 bis 60, und für die 2. Kolonne bei 0,5 bis 10, vorzugsweise bei 1 bis 5, liegen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 340 324 (UNION RHEINISCHE BRAUNKOHLEN KRAFTSTOFF) * Seite 4, Spalte 6, Zeile 58 - Seite 6, Spalte 9, Zeile 21; Seite 7, Spalte 12, Zeile 7 - Seite 8, Spalte13, Zeile 34; Patentansprüche * --- | 1-5 | C07C43/04 C07C41/42 C07C41/09 |
| D,A | DD-A-282 909 (UNION KRAFTSTOFF) * Ansprüche; Abbildungen * ----- | 1-5 | |
| | | | **RECHERCHIERTE SACHGEBIETE (Int. Cl.5)** |
| | | | C07C |

Der vorliegende Recherchenbericht wurde für alle Patentansprüche erstellt

| Recherchenort | Abschlußdatum der Recherche | Prüfer |
|---|---|---|
| DEN HAAG | 16 JUNI 1993 | WRIGHT M.W. |